# EUROPEAN PATENT APPLICATION

(11) **EP 4 376 017 A1**
(43) Date of publication of application: **29.05.2024**
(21) Application number: 22213259.9
(22) Date of filing: 13.12.2022
(51) Int. Cl.: G16H 20/40, G06N 3/08, G06N 20/00, G16H 40/20, G16H 40/63

(54) **AI MODEL OPTIMIZATION THROUGH USER FEEDBACK**

(30) Priority: 28.11.2022 US 202263428154 P
(71) Applicant: Koninklijke Philips N.V., 5656 AG Eindhoven (NL)
(72) Inventor: KOKER, Ekin, Eindhoven (NL); STAROBINETS, Olga, Eindhoven (NL); UHLEMANN, Falk, 5656AG Eindhoven (NL); CHADUVULA, Siva Chaitanya, Eindhoven (NL); WANG, Jessica, Eindhoven (NL); TELLIS, Ranjith Naveen, Eindhoven (NL)
(74) Representative: Philips Intellectual Property & Standards

(57) **Abstract**

A means for obtaining optimized feedback from users of a radiology system for use in continuous training/updating of AI models associated with operations of the radiology system. Information about a current or future status of a user (e.g. availability status, and/or emotional status) and about current model update requirements for one or more AI models is used to determine an optimal user to query for feedback, and an optimal set of feedback items to include in a feedback request.

## Description

### FIELD OF THE INVENTION

The present invention relates to a method and system for obtaining user feedback for use in supplementary training of one or more AI models.

### BACKGROUND OF THE INVENTION

Artificial Intelligence (AI) algorithms are applied in a variety of contexts.

There is growing adoption of AI in healthcare settings. AI models utilizing one or more AI algorithms can be applied for example in the domains of triage, diagnosis and of prediction of disease onset.

One context in which AI models may provide advantage is in radiology. In this context, AI models can perform functions such as: detecting patient pose within acquired imagery of a scanner; tracking parts of scanning devices; detecting presence of persons in a room; matching patients to clinicians; scan protocol planning; anatomical segmentation, and many more.

Accuracy and reliability of AI model outputs is of critical importance in healthcare. Ongoing training of models based on feedback from clinical users is one way of improving accuracy.

Improvements in the area of AI model continuous learning would be of benefit.

### SUMMARY OF THE INVENTION

The invention is defined by the claims.

The inventors have recognized that continuous model learning/updating depends upon obtaining regular and reliable user feedback which can be used as ground truth information for supplementary training of models. However, the inventors have further recognized that soliciting feedback from users is a challenge. The users whose feedback is required are clinicians with patient care responsibilities. The process for obtaining feedback should ideally be sensitive to operational workflows of the users. For example, it should take place in a way which does not interrupt workflow, for instance generating feedback requests at inappropriate times or creating general feedback fatigue for users.

Finding the optimal time window for engaging with the user is important.

The inventors have proposed a method for integrating AI model feedback acquisition within a radiology platform so that information available to the platform can be used to obtain required feedback data most efficiently and effectively for updating AI models. Consequently, this enables a more efficient execution of the overall clinical workflow in a way that integrates targeted feedback collection.

According to examples in accordance with an aspect of the invention, there is provided a system, comprising: a processing device comprising one or more processors; a user interface operatively coupled with the processing device; a radiology apparatus operatively coupled with the processing device, adapted to perform radiology operations; and one or more AI models, each configured to generate an output for use in operation of the radiology apparatus.

The one or more processors may include a model feedback optimization module and a feedback collection module.

The model feedback optimization module is configured to: access a database (or other means of user status information) of available users to query for user input to be used as feedback for supplementary training of at least one of the one or more AI models; obtain information about a current or future status (e.g. availability status or emotion/mood status) of at least a subset of the users in the database; and determine AI model update requirements for one or more of the AI models, the update requirements corresponding to ground truth information for use in supplementary training of one or more of the AI models.

The feedback collection module is configured to generate a feedback request for communication to at least one of the available users, the feedback request including one or more feedback items, each feedback item corresponding to a request for ground truth information for use in supplementary training of one or more of the AI models, and configured to receive user input provided by the at least one of the available users responsive to the feedback request.

The one or more feedback items included in the feedback request may be selected (for example by the feedback optimization module) based at least in part on the identified AI model update requirements. Furthermore, the one or more users to whom the feedback request is communicated may be determined (for example by the feedback optimization module) based at least in part on the status of the at least subset of users in the database.

Embodiments of the invention thus aim to provide optimization of AI models in a way that minimizes disruption of existing radiology workflows. Rather than simply prompting every user for feedback for every model, it is proposed to selectively match particular users to particular model feedback items based on: user status (e.g. availability, busyness) and model update requirements. This provides for more effective updating of the models, since the feedback is obtained from an optimal selection of users (e.g. users who are most technically competent to provide the feedback, users who have the time to provide accurate feedback). At the same time, it also avoids deterioration of clinical care, since the system avoids querying users for feedback during times where they are occupied with aspects of imaging, diagnostic and care-giving workflows.

The optimization module may run repeatedly at regular intervals and determine at the time of each run the optimal users (if any) to send feedback requests to at that time, and the optimal feedback items to include in those requests. Additionally or alternatively, the module may determine an optimal time (in the future) to send feedback requests to at least a subset of users, for instance those users for whom the current time is not optimal.

The feedback optimization module may thus be configured to generate as an output a list of one or more users to be queried for feedback, and, for each user, a list of one or more feedback items to be included in the feedback request communicated to that user. Optionally, it may also be configured to indicate an optimal timing for generating the feedback request for each user, and/or an optimal timing for each feedback item in the feedback request. The feedback collection module may be configured to receive this information as its input and to generate the feedback requests in accordance with the information, either at a current moment or scheduled in the future.

In some embodiments, the generating the feedback request may comprise sending an electronic message to a computing device of the user, e.g. a mobile computing device.

In some embodiments, the model feedback optimization module may execute in real time during control of the radiology apparatus by the user via the user interface.

In some embodiments, each feedback item may comprise a request to the user for ground truth information for use in comparison against generated outputs of one of the AI models, or for generating annotation metadata for appending to training data entries for use in training.

In some embodiments, the system further comprises a model update module configured to perform supplementary training of one or more of the AI models based at least in part on the received user input. For example, this may comprise using the received user input as ground truth information for use in training the models. In practice a range of different sources of ground truth information may be used in performing the supplementary training, of which the received user feedback information is just one element.

In some embodiments, the information about a current or future status of at least a subset of the users in the database may include information about a status of the users as a function of time (e.g. time of day).

In some embodiments, the information about a current or future status of at least a subset of the users in the database is or comprises information about a current or future availability status of the at least subset of the users, in particular an availability status for providing feedback. In some embodiments, the information about a user status (current or future) may be obtained based on accessing a user schedule stored by the processing device. In some embodiments, the information about a user status may be obtained based on processing camera data of the user. In some embodiments, the information about a user status may be obtained based on questionnaire data provided by the user and stored in an accessible database.

In some embodiments, the system is further configured to determine a timing for triggering the generation of the feedback request. For example, this may be based on the current or future status of the at least subset of users (e.g. how busy each user is), or based on user schedule information for a period of time extending into the future. Additionally or alternatively, it may be based on past responses as a function of time of each of the at least subset of users (e.g. probability of response as a function of time of day or week, based on historical response rate as a function of time of day or week). In this way, the feedback requests can be optimally timed to improve probability of response, and also to reduce likelihood that the request interrupts another part of the clinical workflow in which a user is engaged.

In some embodiments, the determining the one or more users to whom the feedback request is communicated may be further based on feedback requirements in relation to a type of user needed for feedback provision. Here, the model feedback requirements may include a required type of clinician user, or a required expertise level of the user to provide the feedback.

Additionally or alternatively, in some embodiments, the determining the one or more users to whom the feedback request is communicated may be further based on user past responses to feedback requests. For example, it may be based on information regarding types of feedback each user has provided in the past. There may be statistical information associated with each user pertaining to likelihood of responding to requests for different feedback items. Thus here, the aim is to identify individuals who have a higher likelihood of providing feedback based on their past behaviors, e.g. as stored in a log.

In some embodiments, the obtaining the information about a current or future status of the at least one of the users includes determining a metric of current or future user busyness based on accessing a user schedule stored by the processing device.

In some embodiments, the obtaining the information about a status of the at least one of the users includes accessing a usage log stored by the processing device to determine a current user usage of the radiology apparatus.

In some embodiments, the obtaining the information about a status of the at least one of the users comprises accessing real-time camera data and/or audio voice data, processing the camera data and/or audio voice data with a scene recognition algorithm and/or speech recognition algorithm to identify whether the user is currently engaged with a patient. In some embodiments, an availability metric of the user might be rated lower if the user is engaged with patient. In some embodiments, camera data or voice data might additionally or alternatively be used to identify user interactions with the user interface, i.e. whether the user is actively engaged with the radiology apparatus.

In some embodiments, the obtaining the information about a status of the at least one of the users includes accessing real-time camera data and/or audio voice data, and processing the camera data and/or audio voice data with an emotion recognition algorithm to determine a user's mood.

With regards to determining the AI model update requirements, in some embodiments, this could be achieved as follows. In some embodiments, each of the one or more AI models may be operable to generate a confidence value indicative of a confidence level of a generated output from the model. Determining the AI model update requirements may then comprise, for at least one AI model, comparing the confidence value against a threshold. This indicates where models have low confidence and so where further training might be beneficial.

In some embodiments, the feedback optimization module may be further configured to: generate a list of candidate feedback items; estimate an impact level of each feedback item on the AI model performance; rank the candidate feedback item(s) according to the estimated impact levels; select one or more of the feedback item(s) based on highest rankings; and wherein the feedback request includes the selected feedback items.

With regards to determining the impact level, various approaches can be used. In some embodiments, this may be based on the aforementioned confidence values associated with different AI model outputs. For example, each of the one or more AI models may be operable to generate a confidence value indicative of a confidence level of a generated output from the model. The method may comprise ranking AI model outputs from one or more AI models according to confidence. Each model output may be associated with a particular feedback item which is known to provide training data related to the model output. The impact level for each feedback item can then be defined based on the confidence level of the associated model output.

In some embodiments, confidence values may be monitored over time, and wherein those model outputs with the most persistently low confidence values are identified. The feedback items which are known to provide training data associated with these model outputs can then be selected as the most impactful feedback items.

In some embodiments, the feedback request includes an option for a user to either decline to provide feedback or to provide feedback. In some embodiments, the system in this case may include a log recording user past choices to either provide or decline feedback. The feedback optimization module may be configured to access said log, and wherein the selection of a user to query for feedback is based in part on the log records. This may allow the system to assess likelihood that a user will be willing to provide feedback in response to a certain feedback request.

In some examples, the log may record timings of each feedback request along with the user choice to decline or to provide feedback, and wherein selection of a timing of a feedback request is based on said timings in the log. In other words, the system may select a timing which correlates with timings for which a user more often provides the feedback rather than declines to provide feedback.

In some embodiments, the feedback optimization module may be further configured to determine a user experience level required for a given feedback item; access a database recording an experience level of each of the available users in the database of available users; select a user to query for feedback based on the experience level of the user compared with the experience level required for the given feedback item.

In some embodiments, the system may be further configured to perform a quality check operation in relation to obtained feedback, comprising: sending a feedback request to multiple different users containing the same feedback item; comparing the user inputs provided by the different users for the feedback item; and determining a reliability metric for the feedback based on the degree of agreement between the responses. The reliability metric may then be used to determine whether or not the feedback is to be used in updating the relevant model. The reliability metric may be compared against a threshold to determine this for instance.

In some embodiments, the feedback optimization module is further configured to: access a database recording user preferences as to feedback items; and determine the one or more users to whom the feedback request is communicated and/or the one or more feedback items to include the feedback request in dependence upon the said user preferences.

Another aspect of the invention is a computer-implemented method for use in updating a system, the system comprising: a processing device comprising one or more processors; a user interface operatively coupled with the processing device; a radiology apparatus operatively coupled with the processing device, adapted to perform radiology operations; and one or more AI models, each configured to generate an output for use in operation of the radiology apparatus.

The method may comprise: accessing a database of available users of a system to query for user input to be used as feedback for supplementary training of at least one of one or more AI models; obtaining information about a current or future status of at least a subset of the users in the database; determining AI model update requirements for one or more of the AI models, the update requirements corresponding to ground truth information for use in supplementary training of one or more of the AI models; and generating a feedback request for communication to at least one of the available users, the feedback request including one or more feedback items, each feedback item corresponding to a request for ground truth information for use in supplementary training of one or more of the AI models; and receiving user input provided by the at least one of the available users responsive to the feedback request.

The one or more feedback items included in the feedback request may be selected based at least in part on the identified AI model update requirements. The one or more users to whom the feedback request is communicated may be determined based on at least the current or future status of the at least subset of users in the database.

Another aspect of the invention is a computer program product comprising computer program code configured, when run on a processing device comprising one or more processors, to cause the processing device to perform a method in accordance with any embodiment described in this disclosure.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 outlines steps of an example method in accordance with one or more embodiments of the invention;
Fig. 2 outlines components of an example processing device and system in accordance with one or more embodiments of the invention;
Fig. 3 outlines a processing flow implemented by the processing device in accordance with one or more embodiments of the invention; and
Fig. 4 outlines example inputs to a feedback optimization module according to one or more embodiments of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a means for obtaining optimized feedback from users of a radiology system for use in continuous training/updating of AI models associated with operations of the radiology system. Information about a current or future status of a user (e.g. availability status, and/or emotional status) and about current model update requirements for one or more AI models is used to determine an optimal user and optionally time to query for feedback, and an optimal set of feedback items to include in a feedback request.

Embodiments of the invention relate to updating of artificial intelligence (AI) models.

In the context of this disclosure an artificial intelligence model means a model which comprises one or more machine learning algorithms.

A machine-learning algorithm is any self-training algorithm that processes input data in order to produce or predict output data.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

Within the context of a radiology apparatus or system, examples of AI models that may find beneficial application include for instance detecting patient pose in acquired imagery of a scanner; tracking parts of scanning devices; detecting presence of persons in a room; matching patients to clinicians; scan protocol planning; anatomical segmentation, and many more.

Various such AI models may be hosted by a radiology software platform which is also connected with a radiology apparatus. The various AI models may be deployed by the software platform as part of an imaging and/or diagnostic workflow, e.g. scan planning, organ localization, patient position alignment, scan execution and so on. The various AI models may be deployed by the software platform as part of clinical operations ancillary to imaging and diagnostics such as scan time scheduling, patient consultation scheduling, patient monitoring and so on.

Although AI models are trained for execution of specific tasks in advance of deployment, when implemented in the real world, it is useful and often necessary to perform continuous updating of the models through ongoing model training. In other words, ongoing model learning is implemented so that model performance can be continually improved and also so that model performance can be customized to the particular use patterns associated with a particular system, as well as system type, institution and/or region.

At least one aim motivating the present application is to implement such a process of ongoing AI model training/updating.

It is proposed for the purposes of embodiments of the present application to use information which would be available in the context of a radiology apparatus to assist in the process of AI model updating.

In particular, part of AI model updating requires obtaining user input or feedback which provides ground truth information against which predictions of the model can be compared as part of a supervised training process. Obtaining such information in reality is a technical challenge. It is proposed in accordance with embodiments of the present invention to use information sources associated with a radiology system to aid in the acquisition of user feedback in a way that is most likely to result in accurate responses from users.

By way of illustration, one example implementation according to at least a subset of embodiments might include one or more of the following elements:
collection of real-time information about at least one user: e.g. user interactions with the radiology apparatus, user schedule, user emotional status;
identification of the need for feedback based on e.g. AI model deployment timeline (e.g. how new the model is), AI model confidence scores, extension of the AI model to new use cases/scenarios, discrepancies in inferences derived based on inputs from different data sources;
identification of users who have a higher likelihood of providing feedback based on their past behaviors; optimization of the need for feedback with user's likely willingness to provide feedback at any given time.

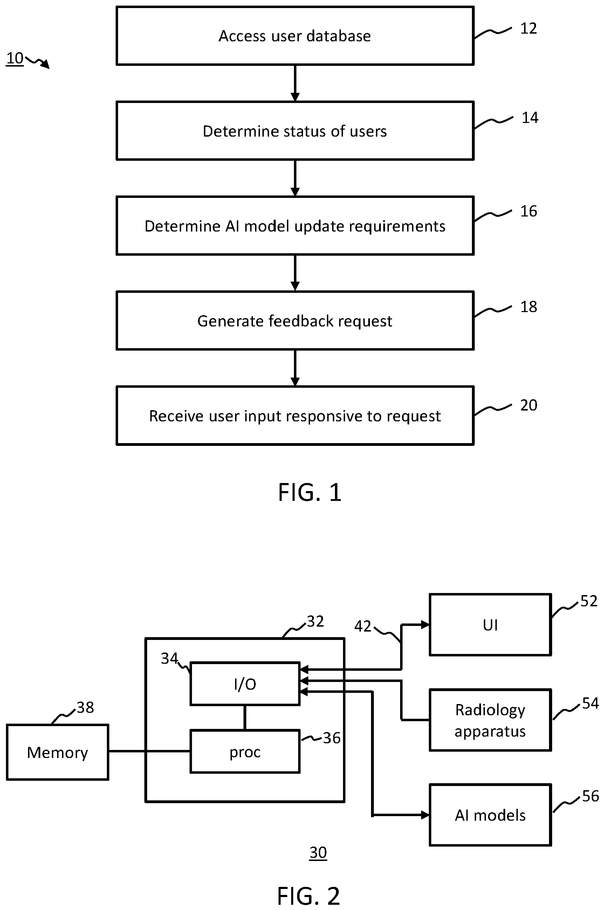

Fig. 1 outlines in block diagram form steps of an example computer implemented method 10 according to one or more embodiments. The steps will be recited in summary, before being explained further in the form of example embodiments.

The method is for use for instance in updating a system. For illustration, Fig. 2 shows components of an example system 30 with which the method might be designed for use. The example of Fig. 2 is illustrative only and the inventive concept is not limited to use with this particular system. The system comprises: a processing device 32 comprising one or more processors 36; a user interface 52 operatively coupled with the processing device; a radiology apparatus 54 operatively coupled with the processing device, adapted to perform radiology operations; and one or more AI models 56, each configured to generate an output for use in operation of the radiology apparatus.

With reference again to Fig. 1, the method 10 comprises accessing 12 a database of available users of the system to query for user input to be used as feedback for supplementary training of at least one of the one or more AI models 56.

The method 10 further comprises obtaining 14 information about a current or future status of at least a subset of the users in the database. The database (not shown in Fig. 2) may be included as a part of the system 30 or might be an external entity with which the processing device is arranged to communicate.

The method further comprises determining 16 AI model update requirements for one or more of the AI models, the update requirements corresponding to ground truth information for use in supplementary training of one or more of the AI models 56.

The method further comprises generating 18 a feedback request for communication to at least one of the available users, the feedback request including one or more feedback items, each feedback item corresponding to a request for ground truth information for use in supplementary training of one or more of the AI models 56.

The method further comprises receiving 20 user input provided by the at least one of the available users responsive to the feedback request.

The aforementioned one or more feedback items included in the feedback request may be selected based on the identified AI model update requirements. The one or more users to whom the feedback request is communicated may be determined based on at least the current or future status of the at least subset of users in the database.

As noted above, the method can also be embodied in hardware form, for example in the form of a processing device 32 which is configured to carry out a method in accordance with any example or embodiment described in this document, or in accordance with any claim of this application. To further aid understanding, Fig. 2 presents a schematic representation of an example processing device 32 configured to execute a method in accordance with one or more embodiments of the invention. The processing device is shown in the context of a system 30 which comprises the processing device. The processing device alone represents an aspect of the invention. The system 30 is another aspect of the invention. The provided system does not have to comprise all of the illustrated hardware components; it may just comprise a subset of them.

The processing device 32 comprises one or more processors 36 configured to perform a method in accordance with that outlined above, or in accordance with any embodiment described in this document or any claim of this application. In the illustrated example, the processing device further comprises an input/output 34 or communication interface.

The system 30 may further comprise a memory 38 for storing computer program code (i.e. computer-executable code) which is configured for causing the one or more processors 36 of the processing device 32 to perform the method as outlined above, or in accordance with any embodiment described in this disclosure, or in accordance with any claim.

In the example of Fig. 2, the system 30 further comprises a user interface 52 operatively coupled with the processing device 32.

In the example of Fig. 2, the system 30 further comprises a radiology apparatus 54 operatively coupled with the processing device 32, adapted to perform radiology operations.

In the example of Fig. 2, the system further comprises one or more AI models, each configured to generate an output for use for example in operation of the radiology apparatus 54. The one or more AI models may be stored on a datastore. Each AI model may comprise one or more machine learning algorithms.

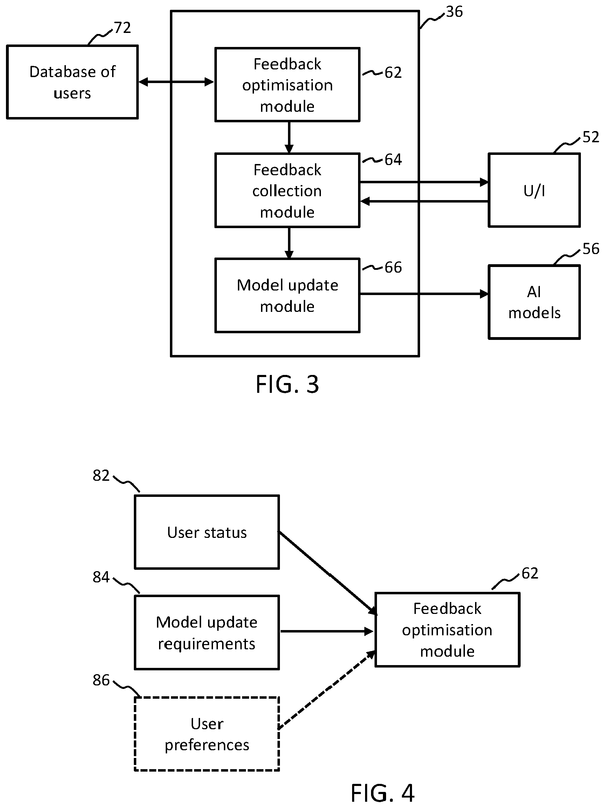

As schematically illustrated further in Fig. 3, in some embodiments, the one or more processors 36 may include a model feedback optimization module 62 and a feedback collection module 64. These may be software modules in preferred cases, or alternatively may be hardware modules. Optionally, in some embodiments, the one or more processors 36 may further comprise a model update module 66 configured to perform supplementary training of one or more of the AI models 56 based at least in part on the received user input.

The model feedback optimization module 62 may be configured to: access a database 72 of available users to query for user input to be used as feedback for supplementary training of at least one of the one or more AI models 56. The model feedback optimization module 62 may be further configured to obtain information about a current or future status of at least a subset of the users in the database 72. The model feedback optimization module 62 may be further configured to determine AI model update requirements for one or more of the AI models 56, the update requirements corresponding to ground truth information for use in supplementary training of one or more of the AI models.

The feedback collection module 64 may be configured to generate a feedback request for communication to at least one of the available users, for example via the user interface 52. The feedback request may include one or more feedback items, each feedback item corresponding to a request for ground truth information for use in supplementary training of one or more of the AI models 56. The feedback collection module 64 may be further configured to receive, e.g. from the user interface 52, user input provided by the at least one of the available users responsive to the feedback request.

The one or more feedback items included in the feedback request may be selected based on the identified AI model update requirements. The one or more users to whom the feedback request is communicated may be determined based on at least the current or future status of the at least subset of users in the database.

The input information to the feedback optimization module 62 is further schematically illustrated in Fig. 4. As discussed above, the feedback optimization module is configured to obtain or determine information about a status 82 of at least a subset of the users in the database 72 and about AI model update requirements 84 for one or more of the AI models 56. As will be explained later, optionally, the feedback optimization module 62 is further configured to determine or obtain information about user feedback preferences 86, e.g. types of feedback item a user would prefer to respond to, or preferred timings of a user for giving feedback.

As mentioned previously, the invention can also be embodied in software form. Thus another aspect of the invention is a computer program product comprising code means configured, when run on a processor, to cause the processor to perform a method in accordance with any example or embodiment of the invention described in this document, or in accordance with any claim of this patent application.

In some embodiments, the one or more processors may host a radiology software platform.

In some embodiments, the user interface 52 of the system 30 may be a user interface which is employed for control and operation of the radiology apparatus 54. For example the user interface may display radiology images generated by the radiology apparatus and may present a graphical user interface presenting control options for user control of the radiology apparatus.

In some embodiments, the same user interface may be used to communicate the feedback request to the user and/or to receive the user input responsive to the feedback request.

In some embodiments, the feedback collection module is configured to transmit the feedback request to a computing device. For example, it may be configured to transmit the feedback request to a mobile or personal computing device associated with the user to whom the feedback request is directed. For example, this might be a smartphone belonging to the relevant user.

In some embodiments, the mobile computing device may host a software application which interfaces with the aforementioned system, optionally via an internet or cloud-based server, and wherein this software app receives feedback requests which have been generated by the feedback collection module 64 directed to the relevant user and wherein the requests are presented to the user via a user interface of the mobile communication device.

Thus, each feedback request generated by the feedback collection module may be tagged with information identifying a target user to whom the request is directed, and with information indicating the one or more feedback items which are part of the request. This feedback request can then be communicated to a computing device associated with the user, or to the user interface 52 of the system, either directly or via an intermediary server such as an internet or cloud-based server.

When the feedback request is presented to the user through the computing device or the user interface 52, the computing device or user interface may permit input by the user of feedback responses to the one or more feedback items. For example this might be text input or pointer input (e.g. through touchscreen presses or a pointer device), audio input (e.g. voice recognition) or simple tactile input (e.g. via buttons). For example, for each feedback item, one or more questions might be presented to the user which prompt for a response. For example, each feedback item presented to the user may include a presentation of the relevant AI model input for which the relevant AI model is adapted to make an output prediction along with a question asking for the ground truth answer (i.e. the correct prediction), or asking for confirmation of an already generated model prediction.

With regards to the feedback items contained in the feedback request, there are a variety of different options. In general, each feedback item comprises a request to the user for ground truth information for use in comparison against generated outputs of one of the AI models as part of a supervised training procedure, or for generating annotation metadata for appending to training data entries for use in a supervised training procedure.

Some illustrative examples will now be outlined. However, the invention is not limited to these examples.

In one example, one of the AI models 56 is a model comprising a machine learning algorithm trained to receive as input an image of the room containing the radiology apparatus 54 and to generate as output an indication of a location in the image of an infusion pump associated with a patient being scanned. In this example, a generated feedback item corresponds to a request for confirmation from the user of a location of the infusion pump. For example, the user is presented with the output from the model indicating the model prediction of the location and the used is asked to click to confirm the location of the infusion pump. Instead of an infusion pump, the same principle can be applied for a model trained to detect a location of any object, including any clinical equipment or apparatus or of the patient or a part of the patient.

In one example, one of the AI models 56 is a model comprising a machine learning algorithm trained to predict from a radiology image a coil type used for the imaging. This can be especially useful for third party systems where there is no access to the system logs. Here, a generated feedback item corresponds to a request for confirmation of the predicted coil type, or for the user to indicate the coil type for use in training.

In one example, one of the AI models 56 is a model comprising a machine learning algorithm trained to predict based on input video data of the radiology apparatus a degree of effort needed to position the patient, e.g. using a discrete scale (e.g. none, moderate, high). Here, the feedback item may correspond to a request for confirmation of the predicted effort, or for input by the user of the needed effort for use as annotation data for training the AI model.

In some embodiments, one of the AI models 56 is model comprising a machine learning algorithm trained to identify objects within an image of the room containing the radiology apparatus, e.g. the patient or portions thereof, or clinical equipment. Here the feedback item may correspond to a request for a user to manually point to or circle regions of an image which contain certain objects. In other cases, the feedback item may correspond to a request for confirmation that certain areas of an image to contain a model-predicted object, e.g. an image is shown with a circled area accompanied by a text label "Is this a patient?". User control options might be presented permitting a user to indicate a response "Yes", "No", "Not sure".

In some embodiments, one of the AI models 56 is model comprising a machine learning algorithm trained to receive as input video imagery of a room containing the radiology apparatus and to classify segments of the video corresponding to particular workflow steps, such as preparation, scanning, cleaning and so on. In this case, the feedback item might include a presentation of the video segment and a request for the user to indicate the relevant step, or to confirm a prediction of the AI model.

In some embodiments, and as briefly mentioned above, the system further comprises a model update module 66 configured to perform supplementary training of one or more of the AI models based on the received user input. For example, the model update module 66 may be configured to implement supervised training of one or more machine learning algorithms comprised by an AI model. The user input provided by the at least one available user in response to the feedback request can be used in the training. For example, the user input is provided as ground truth, and a training operation can then be performed in the usual manner in which the weightings of the model are updated based on comparison of the output with the ground truth answer and minimizing the loss function

In particular, as mentioned above, typically, training of a machine learning algorithm comprises obtaining a training dataset, comprising training input data entries and corresponding training output data entries. The initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

In the context of the present application, it is proposed that the user inputs in response to the feedback requests are used as the aforementioned training output data entries against which the predicted output data can be compared.

Example implementation features of the method according to one or more particular embodiments will now be described by way of illustration of the above-summarized concepts. It will be appreciated that not all features described below are essential to the inventive concept, but are presented as advantageous options which may be utilized.

As already discussed, an important part of the proposed concept is optimizing the model feedback requests to improve the quality of obtained feedback, improve the likelihood of receiving a user response, and to minimize interference with the existing clinical workflow, for example associated with operation of the radiology apparatus.

In this regard, in some embodiments, the method may further comprise determining a timing for triggering the generation of the feedback request.

This may for example be based on the current or future status of the at least subset of users, e.g. current or future availability, busyness, emotional state. The status may be determined based on data such as user schedule data, image data of the user, user questionnaire data. The status of a user may be represented by a numerical score along a scale or by a classification. The status may be computed by a status evaluation algorithm which receives as input the schedule data, image data or questionnaire data (or any other relevant data) and generates as output a classification of status or a status at a future time point, or a predicted status as a function of time over a time period extending into the future. The status evaluation algorithm might be comprised as part of the feedback optimization module.

In some embodiments, the determining the optimal timing of the requests might be based in part on past user responses as a function of time of each of the at least subset of users, e.g. user response rate to requests as a function of request timings.

The determination of the timing of the feedback requests might be performed by the feedback optimization module 62 for example.

To explain further, in order to engage a user at an optimal time, it is useful to have certain information about the user's current or future status, e.g. their availability, their busyness, and/or optionally their emotional state, e.g. upset, overwhelmed.

Examples of ways in which user status could be derived from the system 30 include: assessing how busy or light the day is for the user, for example based on available schedules for the user and/or based on a generic busyness pattern stored by the system indicative of typical busyness as a function of time over a day or a week. In other words, here the obtaining the information about a status of the at least one of the users might involve determining a metric of user busyness, for example based on accessing a user schedule stored by the processing device 32.

Another way to assess user status is to assess patterns of user interactions with the radiology apparatus or with the system as a whole. For example, because the optimization module has access to the radiology apparatus, it can be determined if the user is currently operating the radiology apparatus and, if so, what operations the user is performing. For example, if the expert user is simultaneously supporting three scanners or the user is actively coaching a local technologist, then the user busyness is high. In other words, here the obtaining the information about a status of the at least one of the users might involve accessing a usage log stored by the processing device 32 to determine a current user usage of the radiology apparatus.

Another way to assess user status is to assess patterns of user interactions with patients using cameras or user interactions with the radiology apparatus using usage log information obtained from the radiology apparatus. For example, if a user is in consultation with a patient, then the user availability is low and it may not be appropriate to query that user for feedback at that time. On the other hand, if a user has finished planning or prescribing a radiology scan and the scan is in the process of running through automatic operation, the user busyness is lower, and this might be a good time to engage the user.

In other words, here, the obtaining the information about a current status of the at least one of the users might involve accessing real-time camera data and/or audio voice data, and processing the camera data and/or audio voice data with a scene recognition algorithm and/or speech recognition algorithm to identify whether the user is currently engaged with a patient. In some examples, the method may comprise weighting an availability or busyness metric of the user lower if engaged with patient.

With regards to the scene and/or speech recognition algorithm, the following paper describes suitable techniques from the field of Human Activity Recognition: Khan IU, Afzal S, Lee JW. Human Activity Recognition via Hybrid Deep Learning Based Model. Sensors. 2022; 22(1):323. https://doi.org/10.3390/s22010323.

Another way to assess user status is to assess a user's mood based on camera footage, based on voice patterns, based on contextual factors (e.g. when a user is being observed by his superiors or is dealing with a difficult patient, running behind schedule, or is handling an unfamiliar protocol). In general, if a user is in a low mood, e.g. stressed, or unhappy, the probability of the user responding to a feedback request is low, or the feedback provided may be of poor reliability.

In other words, here the obtaining the information about a current status of the at least one of the users might involve accessing real-time camera data and/or audio voice data, processing the camera data and/or audio voice data with an emotion recognition algorithm to determine a user's mood. In this regard, reference is made to the following paper which describes at least one suitable algorithm for detecting user mood or emotional status based on camera data: Y. Miyakoshi and S. Kato, "Facial emotion detection considering partial occlusion of face using Bayesian network," 2011 IEEE Symposium on Computers & Informatics, 2011, pp. 96-101, doi: 10.1109/ISCI.2011.5958891.

Solicitation for feedback received at an inappropriate time may exacerbate the feelings of stress of a user or lead to feedback fatigue. However, if feedback requests are timed to coincide with a period of lower busyness, there is greater likelihood of a response.

In some embodiments, the method comprises determine a timing for triggering the generation of the feedback request based on past responses as a function of time of each of the at least subset of users. For example, the system may store a log which records timings of historical feedback request along with an indication as to whether the user responded to the request. A selection of a timing of a new feedback request may be based on said timings in the log. For example, in some embodiments, the system might store a probability distribution for each user indicating likelihood of response as a function of time based on historical response patterns. The optimization module may include a response likelihood evaluation algorithm configured to process historical information in the aforementioned log and to generate a probability distribution for each user indicating likelihood of response as a function of time. From this, an optimal timing for generating a request for a given user might be selected.

For example, by referencing a user schedule, the optimization module may determine a given day for which a busyness of the user is below a threshold. By further referencing the historical response pattern information, the optimization module may determine a time during the day where the user is most likely to respond to a feedback request.

As discuss above, in addition to determining a status of one or more users, the optimization module is further configured to determine AI model update requirements for one or more of the AI models.

Querying users too frequently for model feedback can lead to users experiencing 'feedback fatigue' which could lead to complete disconnect from the platform and user frustration. Therefore it is beneficial to be more selective about the feedback that is requested. For example, it is helpful to narrow down which specific AI models are most in need of supplementary training and/or the training data which would be most impactful. If user feedback is likely to improve algorithm accuracy only marginally, e.g. from 95.6 to 95.8, it may be prudent to reserve user feedback for a problem where a more significant impact is anticipated.

Thus, it is proposed for the optimization algorithm to track the feedback requirements of the one or more AI models.

One example way of tracking feedback requirements of an AI model is to evaluate confidence values associated with the model predictions. Another example way of tracking feedback requirements of an AI model is to query human developers for this information.

For example, each of the one or more AI models may be operable to generate a confidence value indicative of a confidence level of a generated output from the model. In some embodiments, the determining the AI model update requirements comprises, for at least one AI model, comparing the confidence value against a threshold. For example, if confidence is below a threshold, then this indicates that training would be beneficial to improve the particular prediction type to which generated output corresponds.

In some embodiments, the feedback optimization module may be further configured to: generate a list of candidate feedback items; estimate an impact level of each feedback item on the AI model performance; rank the candidate feedback items according to the estimated impact levels; and select one or more of the feedback item(s) based on highest rankings. The feedback request generated by the system may then include the selected feedback items. This is a way of ensuring that the feedback requests pertain to feedback items which will have the greatest impact.

With regards to determining the impact level, various approaches are possible. In some embodiments, this could be based on the aforementioned confidence values associated with different AI model outputs. For example, each of the one or more AI models may be operable to generate a confidence value indicative of a confidence level of a generated output from the model. The method may comprise ranking AI model outputs from one or more AI models according to confidence. Each model output may be associated with a particular feedback item which is known to provide training data related to the model output. The impact level for each feedback item can then be defined based on the confidence level of the associated model output.

In some embodiments, confidence values may be monitored over time, and wherein those model outputs with the most persistently low confidence values are identified. The feedback items which are known to provide training data associated with these model outputs can then be selected as the most impactful feedback items.

As mentioned above, in some embodiment it is proposed to keep track of user choices as to whether or not to respond to requests for feedback. This information may be used by the optimization module in determining timings for generating feedback requests for particular users. For example, if a particular user consistently rejects feedback prompts on every occasion apart from Wednesday mornings, then Wednesday mornings might be the best time window in which to send feedback requests. Additionally, identifying the type of engagement preferred by the user may also help in increasing engagement.

Thus, in some embodiments, each feedback request generated by the system may include an option for a user to either decline to provide feedback or to provide feedback. The system may include a log recording user past choices to either provide or decline feedback. The feedback optimization module may be configured to access said log. The selection of a user to query for feedback may then be based in part on the log records. Users who are more likely in general to respond may be better to query. In addition, as mentioned above, a timing for a feedback request for a particular user may be determined by the optimization module based on the log. For example, the log might record timings of each feedback request along with the user choice to decline or to provide feedback, and wherein selection of a timing of feedback request is based on said timings in the log.

As mentioned above, it is also beneficial to take into account user preferences.

Thus, in some embodiments, the feedback optimization module is adapted to access a database recording user preferences as to feedback items and to determine the one or more users to whom the feedback request is communicated and/or the one or more feedback items to include the feedback request in dependence upon the said user preferences.

Another possible factor to take into account in optimizing the feedback requests is the particular experience patterns and levels of different users (e.g. senior user, multi-modality expert). Depending upon the particular task which an AI model is trained to perform, the input ground truth data required to train that model may need to come from human users of different experience or expertise levels.

Thus, in some embodiments, the feedback optimization module is further configured to:
determine a user experience level required for a given feedback item (this might be recorded in a database for example); access a database recording an experience level of each of the available users in the database of available users; and select a user to query for feedback based on the experience level of the user compared with the experience level required for the given feedback item.

In some embodiments, the determining the one or more users to whom the feedback request is communicated may be based in part on feedback requirements in relation to type of user needed for feedback provision.

Another consideration in optimizing feedback requests is the need for obtained user input to provide reliable and trustworthy ground truth information. If the obtained user input is of poor accuracy, then model performance will deteriorate.

One way of assessing this is to implement functionality for ranking the feedback of different users based on comparing the feedback of different users to the same prompt questions. For example, the same feedback items may be included in feedback requests of multiple different users and for example the majority outcome may be considered correct.

As a more general principle, according to one or more embodiments, the system may be further configured to perform a quality check operation in relation to obtained feedback, comprising: sending a feedback request to multiple different users containing the same feedback item; comparing the user inputs provided by the different users for the feedback item; and determining a reliability metric for the feedback based on the degree of agreement between the responses.

Another way of assessing reliability of feedback is to include in the feedback request prompts for responses to test questions to which reliable answers are already known, and comparing the user's responses to the test questions against the known answers. If the user's responses differ from the known answers, then the user's feedback may not be reliable.

For example, if an AI model is configured for predicting room occupancy based on image data of a room, a feedback request might comprise presenting a user with a plurality of images, and prompting for user input indicating occupancy or non-occupancy for each image, and wherein, for at least a subset of the images, reliable answers are already known. For example, if a room occupancy status algorithm has low confidence that the scanner is occupied by a patient, the system can get feedback from a user by presenting this image along with other images for which high confidence occupancy predictions have already been generated. This approach is hence analogous to CAPTCHA prompts.

As discussed above, an aim is to improve efficiency of feedback collection by timing feedback requests as far as possible so that they do not coincide with busy times of a user. However, some feedback requests may relate to real activities or interactions between the user and the system. When the feedback request is presented to the user at a non-busy time, they may have forgotten the context of the scenario. Thus, in some embodiments, a feedback request may comprise a prompt for user input in relation to an earlier user-system interaction, and wherein the feedback request includes a visual representation of the user-system interaction. For example this may comprise capturing of information related to different user-system interactions for use in later feedback requests (e.g. workflow steps, screenshots, task logs). The communication of the feedback request to the user may then include presenting/re-playing the information to the user. This increases overall efficiency and optimizes user memory recall compared to other solutions.

By way of one example, if feedback is requested for a situation which took place three hours ago (but due to the user being busy it could not be requested earlier) the same situation might be "replayed", e.g. a screen shot of the relevant MRI survey images shown to the user, to thereby remind the user of the scenario. For example, the requested feedback might include a request for confirmation of a patient positioning prediction.

As discussed above, embodiments of the invention involve generating feedback requests for communication to at least one of the available users, the feedback request including one or more feedback items, each feedback item corresponding to a request for ground truth information for use in supplementary training of one or more of the AI models and receiving user input provided by the at least one of the available users responsive to the feedback request. The communication of a given feedback request may be performed through different feedback collection channels, meaning that feedback requests may be communicated to users through different communication media. In some embodiments, at least a subset of feedback requests are communicated to users via the user interface 52 of the system. In some embodiments, at least a subset of feedback requests are communicated to users via a personal computing device of the user such as a smartphone.

With regards to the feedback requests, these may communicated to a user through a variety of different user-perceptible prompts such as multiple choice questions, audio prompts, image prompts.

Feedback requests may be communicated to a same user at different time points throughout a day. As discussed above, these time points may be customized for a given user based on prior response rates for a user as a function of time. Additionally or alternatively, the time points may be pre-determined times defined relative to certain events, such as after a radiology exam has ended, or during scheduled staff breaks.

In some embodiments, a log may be stored in a datastore and the system may be adapted to record response rates for different users. This would allow radiology departments to track different users' response rates and potentially to incentivize providing feedback by factoring those response rates into individual key performance indicators.

Thus, in summary, the feedback optimization module may be configured to receive or to derive as input: information about a status of a user (at a current time point or at a future time point, or as a function of time) and AI model update requirements of one or more AI models.

The feedback optimization module may be configured to generate as an output a list of one or more users to be queried for feedback, and, for each user, a list of one or more feedback items to be included in the feedback request communicated to that user. It may also be configured to indicate an optimal timing for generating the feedback request for the user, and/or an optimal timing for each feedback item in the feedback request.

With regards to the implementation of the feedback optimization module, in order to generate the outputs based on the inputs, there are different options for implementing this.

In a simple implementation, one or more rules based algorithm could be used, wherein rules are defined in advance relating to the various inputs, and wherein a certain feedback item is communicated to a certain user dependent upon the different rules being met.

For example, an algorithmic decision tree could be used to determine which user(s) to query for feedback, and which feedback items to include in the feedback request to the user.

For instance one illustrative example of a decision tree might be as follows:
Only ask a given user for feedback during time T if
   time T is NOT within pre-defined "do not disturb" hours (e.g. provided by user at some time beforehand); Detected stress level of user at time T is below a threshold (e.g. as determined from image analysis of from analysis of user schedule, e.g. light schedule);
A pre-defined minimum experience level needed for AI model training is met by given user experience level.

Another way to implement the decision making of the feedback optimization module is to use one or more machine learning algorithms to perform the determination of the optimal feedback requests, optimal user to query with the feedback request and preferably optimal timing for the feedback request.

For example, a classification model may be provided, for example comprising one or more Logistic regression algorithms, Random Forest classifiers, or support vector machines, trained to provide a prediction as to whether a user will provide a response to a feedback request for a particular feedback item at a particular time based on the various input features already described above. For example, and by way of brief reminder, these input features may include: a current or future busyness level of the user (e.g. quantified or classified, e.g. very busy, busy, passive activity, free), a user's schedule (e.g. pre-classified as overloaded, manageable, light), user operations status (e.g. classified as running late, on time, ahead of time), user feedback preferences (e.g. medium of feedback response, e.g. gaming, text questions, video recordings), user preference as to types of AI model for which to provide feedback (e.g. scan control, patient scheduling, patient consent).

Some of these features are based on a current user workflow and others based on scheduled future workflow, and others based on processing of historical data.

When the system detects a need for AI model updating, the classification model may be executed at regular intervals until to determine which of the current users are most likely to provide feedback at that time.

In some embodiments, the optimization algorithm may employ use of a mixture of rules-based algorithms and machine learning algorithms.

In some embodiments, the optimization module may comprise a plurality of individual algorithms, some of which may be rules-based algorithms, and some of which may be machine learning algorithms, and wherein each individual algorithm is configured to perform a pre-defined data classification task. These various algorithms can be structured to process an overall set of input data through various processing branches to arrive at an output list of optimal users to query for feedback, optimal feedback items for each user, and optionally an optimal time for generating each feedback request. By way of example, one processing branch might include a first algorithm configured to receive as input user schedule data for a certain period of time, and to generate as output a classification of user busyness level as a function of time over that period, or to generate as output a classification of user busyness at a current time. The output of this algorithm could then be supplied as one of a set of inputs to a further classification algorithm which determines an optimal time to generate a feedback request for a user. Thus, it is to be recognized that the various determinations made by the optimization algorithm can be performed through a plurality of classification or prediction algorithms, rather than only a single algorithm.

Furthermore, it is to be noted that the output of the optimization module may vary in different embodiments. In some embodiments, the output of the optimization module is a list indicating a predicted optimal set of users to query for model feedback at a certain moment in time, along with a set of optimal feedback items to include in feedback requests for those users. In some embodiments, this list may further include an indication of an optimal time at which to generate the feedback request for each user. Alternatively, the optimization module may simply be run recurrently at a repeated time points, and wherein the output list always relates to an optimal set of users to query at a current moment in time.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system, comprising:
a processing device (32) comprising one or more processors (36);
a user interface (52) operatively coupled with the processing device;
a radiology apparatus (54) operatively coupled with the processing device, adapted to perform radiology operations;
one or more AI models (56), each configured to generate an output for use in operation of the radiology apparatus; and
wherein the one or more processors include a model feedback optimization module (62) and a feedback collection module (64);
wherein the model feedback optimization module (62) is configured to:
access a database (72) of available users to query for user input to be used as feedback for supplementary training of at least one of the one or more AI models (56);
obtain information about a current or future status of at least a subset of the users in the database;
determine AI model update requirements for one or more of the AI models, the update requirements corresponding to ground truth information for use in supplementary training of one or more of the AI models; and
wherein the feedback collection module (64) is configured to generate a feedback request for communication to at least one of the available users, the feedback request including one or more feedback items, each feedback item corresponding to a request for ground truth information for use in supplementary training of one or more of the AI models, and configured to receive user input provided by the at least one of the available users responsive to the feedback request;
wherein the one or more feedback items included in the feedback request is selected based at least in part on the identified AI model update requirements;
wherein the one or more users to whom the feedback request is communicated is determined based at least in part on at least the current or future status of the at least subset of users in the database.

2. The system of claim 1, wherein the system further comprises a model update module (66) configured to perform supplementary training of one or more of the AI models based on the received user input.

3. The system of claim 1 or 2, wherein the model feedback optimization module is further configured to determine a timing for triggering the generation of the feedback request, for example based on the current or future status of the at least subset of users and optionally based on past response rate as a function of time of each of the at least subset of users.

4. The system of any of claims 1-3, wherein the determining the one or more users to whom the feedback request is communicated is further based on: feedback requirements in relation to a type of user needed for feedback provision.

5. The system of any of claims 1-4, wherein each feedback item comprises a request to the user for ground truth information for use in comparison against generated outputs of one of the AI models, or for generating annotation metadata for appending to training data entries for use in training.

6. The system of any of claims 1-5, wherein the obtaining the information about a current or future status of the at least one of the users includes:
determining a metric of current or future user busyness based on accessing a user schedule stored by the processing device;
accessing a usage log stored by the processing device to determine a current user usage of the radiology apparatus;
accessing real-time camera data and/or audio voice data, processing the camera data and/or audio voice data with a scene recognition algorithm and/or speech recognition algorithm to identify whether the user is currently engaged with a patient;
accessing real-time camera data and/or audio voice data, processing the camera data and/or audio voice data with an emotion recognition algorithm to determine a user's mood.

7. The system of any of claims 1-6,
wherein each of the one or more AI models is operable to generate a confidence value indicative of a confidence level of a generated output from the model; and
wherein the determining the AI model update requirements comprises, for at least one AI model, comparing the confidence value against a threshold.

8. The system of any of claims 1-7, wherein the feedback optimization module is further configured to:
generate a list of candidate feedback items;
estimate an impact level of each feedback item on the AI model performance;
rank the candidate feedback items according to the estimated impact levels; and
select one or more of the feedback items based on highest rankings;
wherein the feedback request includes the selected feedback items.

9. The system of any of claims 1-8,
wherein the feedback request includes an option for a user to either decline to provide feedback or to provide feedback;
wherein the system includes a log recording user past choices to either provide or decline to provide feedback, and wherein the feedback optimization module is configured to access said log;
wherein the selection of a user to query for feedback is based in part on the log records.

10. The system of claim 9 and claim 3, wherein the log records timings of each feedback request along with the user choice to decline or to provide feedback, and wherein selection of a timing of a feedback request is based on said timings in the log.

11. The system of any of claims 1-10,
wherein the feedback optimization module is further configured to
determine a user experience level required for a given feedback item;
access a database recording an experience level of each of the available users in the database of available users; and
select a user to query for feedback based on the experience level of the user compared with the experience level required for the given feedback item.

12. The system of any of claims 1-11, wherein the system is further configured to perform a quality check operation in relation to obtained feedback, comprising:
sending a feedback request to multiple different users containing the same feedback item;
comparing the user inputs provided by the different users for the feedback item; and
determining a reliability metric for the feedback based on a degree of agreement between the responses.

13. The system of any of claims 1-12, wherein the feedback optimization module is further configured to:
access a database recording user preferences as to feedback items;
determine the one or more users to whom the feedback request is communicated and/or the one or more feedback items to include in the feedback request in dependence upon the said user preferences.

14. A computer-implemented method for use in updating a system, the system comprising:
a processing device (32) comprising one or more processors (36);
a user interface (52) operatively coupled with the processing device;
a radiology apparatus (54) operatively coupled with the processing device, adapted to perform radiology operations; and
one or more AI models (56), each configured to generate an output for use in operation of the radiology apparatus;
the method (10) comprising:
accessing (12) a database of available users of a system to query for user input to be used as feedback for supplementary training of at least one of one or more AI models;
obtaining (14) information about a current or future status of at least a subset of the users in the database;
determining (16) AI model update requirements for one or more of the AI models, the update requirements corresponding to ground truth information for use in supplementary training of one or more of the AI models;
generating (18) a feedback request for communication to at least one of the available users, the feedback request including one or more feedback items, each feedback item corresponding to a request for ground truth information for use in supplementary training of one or more of the AI models; and
receiving (20) user input provided by the at least one of the available users responsive to the feedback request,
wherein the one or more feedback items included in the feedback request is selected based at least in part on the identified AI model update requirements, and
wherein the one or more users to whom the feedback request is communicated is determined based at least on part on the current or future status of the at least subset of users in the database.

15. A computer program product comprising code means configured, when run on a processing unit comprising one or more processors to cause the processing unit to perform a method in accordance with claim 14.
